(19) Europäisches Patentamt
European Patent Office
Office européen des brevets

(11) EP 1 621 603 A1

(12) **EUROPEAN PATENT APPLICATION**
published in accordance with Art. 158(3) EPC

(43) Date of publication:
**01.02.2006 Bulletin 2006/05**

(21) Application number: **03751398.3**

(22) Date of filing: **09.10.2003**

(51) Int Cl.:
*C11D 1/34* (1985.01) *C11D 1/29* (1985.01)
*C11D 1/90* (1985.01) *C11D 1/92* (1985.01)
*C11D 1/75* (1990.01) *C11D 9/02* (1985.01)
*C11D 1/28* (1985.01) *C11D 1/72* (1990.01)
*A61K 8/30* (0000.00)

(86) International application number:
**PCT/JP2003/012941**

(87) International publication number:
**WO 2004/092315 (28.10.2004 Gazette 2004/44)**

(84) Designated Contracting States:
**DE FR GB**

(30) Priority: **14.04.2003 JP 2003109000**

(71) Applicant: **KAO CORPORATION**
**Tokyo 103-8210 (JP)**

(72) Inventors:
- **FUKUDA, Morinobu**
  **Sumida-ku, Tokyo 131-8501 (JP)**
- **TETSU, Makio**
  **Sumida-ku, Tokyo 131-8501 (JP)**
- **YOKOTSUKA, Dai**
  **Sumida-ku, Tokyo 131-8501 (JP)**
- **KANEKO, Yohei**
  **Wakayama-shi, Wakayama 640-8580 (JP)**
- **SUZUKI, Nobuyoshi**
  **Wakayama-shi,**
  **Wakayama 640-8580 (JP)**

(74) Representative: **HOFFMANN EITLE**
**Patent- und Rechtsanwälte**
**Arabellastrasse 4**
**81925 München (DE)**

# (54) CLEANING AGENT COMPOSITION

(57) The present invention provides a cleanser composition having a high foaming ability with a low stimulus to the skin. The cleanser composition of the present invention is weakly acidic and comprises (a) a phosphate monoester represented by the general formula (1) and (b) a phosphate diester represented by the general formula (2) wherein the ratio of the component (a) to the component (b), i.e. the (a)/(b) ratio, is from 65/35 to 90/10 by weight:

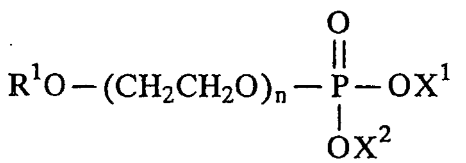

(1)

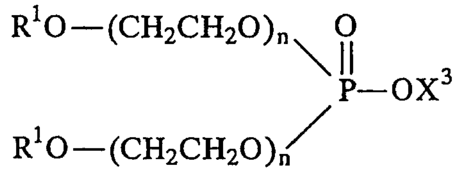

(2)

wherein $R^1$ represents an alkyl or alkenyl group containing 9 to 15 carbon atoms on average with a branching degree of 10% or more, $X^1$ and $X^2$ and $X^3$ each represent a hydrogen atom or an alkali metal, and n is a number of 0 to 5.

EP 1 621 603 A1

## Description

Technical Field

The present invention relates to a cleanser composition having a high foaming ability with a low stimulus to the skin.

Background Art

Conventionally, anionic surfactants such as higher fatty acid salts, alkyl sulfates, polyoxyethylene alkyl sulfates, alkyl benzene sulfonates, and α-olefin sulfonates have been widely used as cleanser for washing the skin. However, when cleanser containing such anionic surfactants are used, there is a tendency to cause skin damages such as chaps on the hand.

On the other hand, nonionic surfactants are known to hardly cause chapped skin, but are inferior to the anionic surfactants in fundamental performance as a cleanser, such as foaming ability and detergency.

As one kind of anionic surfactant, a phosphate-based surfactant is known as a mild surfactant with a low stimulus to the skin. This surfactant is produced, for example, by reacting a fatty alcohol with a phosphorylating agent such as phosphoric anhydride or phosphorus oxychloride, and the reaction product is obtained usually as a mixture of monoesters and diesters or a mixture of mono-, di- and triesters. Particularly, when the resulting alkyl esters contain a higher content of diesters and triesters with a high content of linear chains, they are inferior in solubility in water and foaming ability, and thus the purity of the monoesters should be increased through a troublesome purification process.

On the other hand, a phosphate compound having an oxyethylene group introduced into it for improving water solubility is also known, but this compound is also poor in foaming ability and inferior in performance as a cleanser base material.

To improve such foaming ability, JP-A-2001-181677 proposes a cleanser composition comprising a phosphate-based surfactant having a branched alkyl group, but is still not satisfactory.

Disclosure of Invention

The object of the present invention is to provide a cleanser composition having a high foaming ability with a low stimulus to the skin.

The present invention provides a cleanser composition which is weakly acidic and comprises the following components (a) and (b) in a (a)/(b) ratio of from 65/35 to 90/10 by weight:

(a) a phosphate monoester represented by the general formula (1) or a salt thereof:

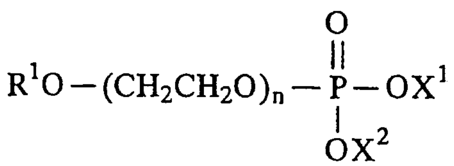

(1)

wherein $R^1$ represents an alkyl or alkenyl group containing 9 to 15 carbon atoms on average with a branching degree of 10% or more, $X^1$ and $X^2$ each represent a hydrogen atom or an alkali metal, and n is a number of 0 to 5 which refers to the number of ethylene oxide units added on average,

(b) a phosphate diester represented by the general formula (2) or a salt thereof:

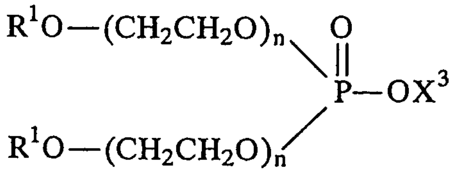

(2)

wherein $R^1$ and n each have the same meaning as defined above, and $X^3$ represents a hydrogen atom or an alkali metal.

Further, the present invention provides a cleanser composition which further comprises at least one co-surfactant (referred to hereinafter as component (c)) selected from the group consisting of an alkyl ethoxylate sulfate, a betaine-type surfactant, a fatty acid or a salt thereof, an amine oxide, an isethionic acid-based surfactant, a sugar-based surfactant, an alkanol amide, an N-acylamino acid salt and an N-acyl-N-methyl taurine salt.

Further, the present invention provides a cleanser composition optionally comprising a $C_{5-6}$ glycol as component (d), which is highly foaming with less increase in viscosity from ordinary temperatures to low temperatures, thus making itself easily handled even at low temperatures.

Further, the present invention provides a weakly acidic cleanser composition optionally comprising as component (e) a thickening polymer compound having a carboxyl group, which gives a freshening feeling in use and is preferable as a cleanser for washing the body and face.

Detailed Description of the Invention

In the components (a) and (b) in the present invention, $R^1$ is an alkyl or alkenyl group containing 9 to 15 carbon atoms on average, preferably 10 to 14 carbon atoms, more preferably 11 to 13 carbon atoms, and having a branching degree of 10% or more, preferably 10 to 60%, from the viewpoint of foaming ability and water solubility.

The branching degree refers to the ratio (weight %) of branched alkyl groups or branched alkenyl groups to all alkyl groups or alkenyl groups represented by $R^1$, and the actual branching degree is determined by analyzing sample by gas chromatography and calculating the branching degree from the peak areas of the corresponding linear esters and branched esters, as shown in the following equation:

$$\text{Branching degree (\%)} = (\Sigma\text{peak areas of branched chains})/(\Sigma\text{peak areas of linear chains} + \Sigma\text{peak areas of branched chains})\times 100$$

$X^1$, $X^2$ and $X^3$ each represent a hydrogen atom or an alkali metal, and examples of the alkali metal include lithium, sodium and potassium, among which sodium and potassium are preferable. n is a number of 0 to 5 indicative of the number of ethylene oxide units added on average, preferably 0 to 3.

The ratio of the component (a) to the component (b) (that is, (a)/(b)) in the cleanser composition of the present invention is 65/35 to 90/10, preferably 65/35 to 85/15 by weight from the viewpoint of water solubility, foaming ability, etc. The total content of the components (a) and (b) in the cleanser composition of the present invention is preferably 3 to 50% by weight, more preferably 5 to 35% by weight.

The components (a) and (b) in the present invention can be obtained as a mixture of the components (a) and (b), for example, by reacting their corresponding fatty alcohols with a phosphorylating agent such as phosphoric anhydride or phosphorus oxychloride under such conditions that the components (a) and (b) are obtained in the weight ratio described above, and then neutralizing the products with an alkali such as sodium hydroxide or potassium hydroxide. By the above reaction, phosphate triesters are also formed, but in the present invention, it is preferable that the content of phosphate triesters in the mixture is 1% by weight or less, that is, the total content of the components (a) and (b) is 99% by weight or more.

The phosphate compound based on the components (a) and (b) has a specific branched structure and oxyethylene groups thereby exhibiting an excellent foaming ability without further purification.

As the fatty alcohols used, a mixture of fatty alcohols that were mixed so as to have the number of carbon atoms on average and the branching degree as defined above, or commercial fatty alcohols having the number of carbon atoms on average and the branching degree as defined above, can be used.

The cleanser composition of the present invention contains the components (a) and (b) in the ratio defined above, and shows weak acidity. The terms "weak acidity" refers to a pH value of 4.5 to 6.5 at 25°C upon dilution of the composition with water. In particular, the cleanser composition of the present invention is preferably the one having a pH value of 4. 5 to 6.5 upon dilution at a concentration of 5% by weight with deionized water.

The pH value of the composition is regulated to be in the above range with an acid or a base, and adjusted preferably to pH 4.5 to 6.0. The organic acid includes, for example, citric acid, succinic acid, lactic acid, malic acid, glutamic acid, aspartic acid, pyrrolidone carboxylic acid, tartaric acid, glycolic acid, ascorbic acid, etc. The inorganic acid includes hydrochloric acid, sulfuric acid, phosphoric acid, etc. Among them phosphoric acid is particularly preferable. As the base, sodium hydroxide or potassium hydroxide are preferable.

The cleanser composition of the present invention further comprises at least one co-surfactant as component (c) selected from the group consisting of an alkyl ethoxylate sulfate, a betaine-type surfactant, a fatty acid or a salt thereof, an amine oxide, an isethionic acid-based surfactant, a sugar-based surfactant, an alkanol amide, an N-acylamino

acid salt and an N-acyl-N-methyl taurine salt. In the cleanser composition of the present invention, the content of the component (c) is preferably 0.5 to 20% by weight, more preferably 1 to 10% by weight.

The co-surfactant as component (c) is preferably at least one member selected from the group consisting of the following (c-1) to (c-9):

(c-1) an alkyl ethoxylate sulfate represented by the general formula (3):

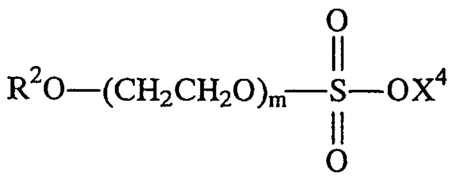

(3)

wherein $R^2$ represents a linear or branched alkyl or alkenyl group containing 10 to 18 carbon atoms on average, $X^4$ represents an alkali metal, and m is a number of 0 to 10 indicating the number of ethylene oxide units added on average.

(c-2) a betaine-type surfactant represented by the general formula (4):

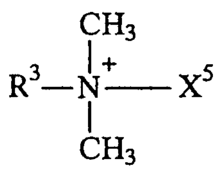

(4)

wherein $R^3$ represents an alkyl or alkenyl group containing 8 to 18 carbon atoms on average or an acyl amino alkyl group represented by the formula $R^4CONH(CH_2)_a$- whereupon $R^4CO$ represents an acyl group containing 8 to 18 carbon atoms on average and a is an integer of 2 to 4, and $X^5$ represents a- $CH_2CH(OH)CH_2SO_3^-$ group or a $-CH_2COO^-$ group.

(c-3) a fatty acid or a salt thereof represented by the general formula (5):

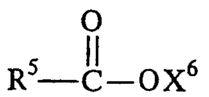

(5)

wherein $R^5$ represents a linear or branched alkyl or alkenyl group containing 9 to 17 carbon atoms on average, and $X^6$ represents a hydrogen atom, an alkali metal, $NH_4$ or alkanol ammonium.

(c-4) an amine oxide represented by the general formula (6):

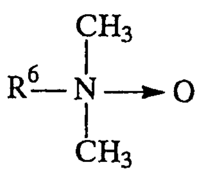

(6)

wherein $R^6$ represents a linear or branched alkyl or alkenyl group containing 8 to 18 carbon atoms on average or an acyl amino alkyl group represented by the formula $R^7CONH(CH_2)_b$- whereupon $R^7CO$ represents an acyl group containing 8 to 18 carbon atoms on average and b is an integer of 2 to 4.

(c-5) an isethionic acid-based surfactant represented by the general formula (7):

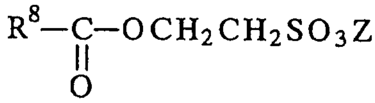 (7)

wherein $R^8$ represents a linear or branched alkyl or alkenyl group containing 9 to 17 carbon atoms on average, and Z represents a hydrogen atom, an alkali metal, $NH_4$ or alkanol ammonium.

(c-6) a sugar-based surfactant represented by the general formula (8):

$$R^9\text{-O-}(R^{10}O)_p\text{- }(G)_q \quad (8)$$

wherein $R^9$ represents a alkyl or alkenyl group containing 8 to 18 carbon atoms on average, $R^{10}$ represents an alkylene group containing 2 to 4 carbon atoms, G represents a residue derived from a reducing sugar containing 5 to 6 carbon atoms, p is a number of 0 to 10 indicating the number of alkylene oxide units added on average, and q is a number of 1 to 10 indicating the average condensation degree of the reducing sugar.

(c-7) an alkanol amide represented by the general formula (9):

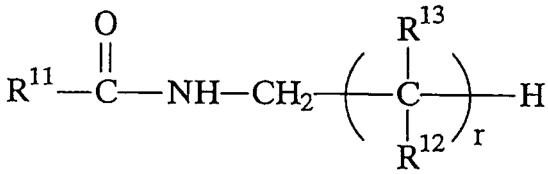 (9)

wherein $R^{11}$ represents a linear or branched alkyl or alkenyl group containing 7 to 17 carbon atoms on average, $R^{12}$ represents a hydrogen atom or a methyl group, $R^{13}$ represents a hydroxyl group or a hydrogen atom, r is a number of 1 to 5, and $R^{12}$ and $R^{13}$ , appearing in the number of r, may be the same as or different from among themselves, provided that at least one of $(R^{13})r$ groups is a hydroxyl group.

(c-8) an N-acylamino acid salt having an acyl group containing 8 to 18 carbon atoms on average,

(c-9) an N-acyl-N-methyl taurine salt having an acyl group containing 8 to 18 carbon atoms on average.

In the alkyl ethoxylate sulfate compound (c-1), $R^2$ is a linear or branched alkyl or alkenyl group containing 10 to 18 carbon atoms on average, preferably 10 to 14 carbon atoms, more preferably 11 to 13 carbon atoms, from the viewpoint of foaming ability. $X^4$ represents an alkali metal. Examples of the alkali metal include lithium, sodium, potassium, etc. Among them sodium and potassium are preferable. m is a number of 0 to 10, preferably 0 to 3, indicating the number of ethylene oxide units added on average.

In the betaine-type surfactant (c-2), $R^3$ represents an alkyl or alkenyl group containing 8 to 18 carbon atoms on average, preferably 10 to 14 carbon atoms, or an acyl amino alkyl group represented by the formula $R^4CONH\ (CH_2)_a$- ($R^4CO$ represents an acyl group containing 8 to 18 carbon atoms on average, preferably 10 to 14 carbon atoms, and a is an integer of 2 to 4, preferably 3) from the viewpoint of thickening properties and foaming ability. $R^3$ and $R^4CO$ may be a mixed alkyl group or an alkenyl or acyl group derived from natural oils, for example, animal oils such as tallow and lard oil or vegetable oils such as soybean oil, coconut oil and palm kernel oil, or synthetic oils and mixed oils thereof. In particular, a mixed alkyl group or an acyl group derived from coconut oil and palm kernel oil is preferable. $X^5$ represents a $-CH_2CH(OH)CH_2SO_3^-$ group or a $-CH_2COO^-$ group.

The betaine-type surfactant (c-2) is preferably hydroxy sulfobetaine wherein $R^3$ is an alkyl or alkenyl group containing 8 to 18 carbon atoms on average, $X^5$ is a $-CH_2CH(OH)CH_2SO_3^-$ group, or amide propyl carboxy betaine wherein $R^3$ is an acyl amino propyl group represented by $R^4CONHC_3H_6^-$ ($R^4CO$ has the same meaning as defined above) and $X^5$ is a $-CH_2COO^-$ group. Examples of the betaine-type surfactant include lauryl dimethyl hydroxy sulfobetaine represented by formula (10), lauroyl amino propyl dimethyl carboxy betaine represented by formula (11), coconut oil fatty acid amide propyl dimethyl carboxy betaine represented by formula (12), etc.

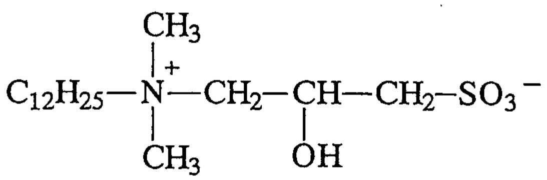

(10)

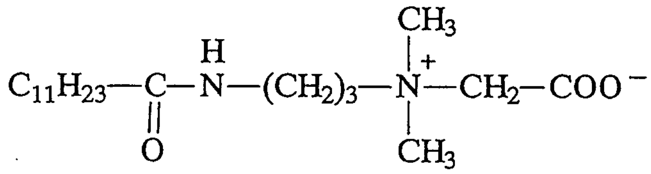

(11)

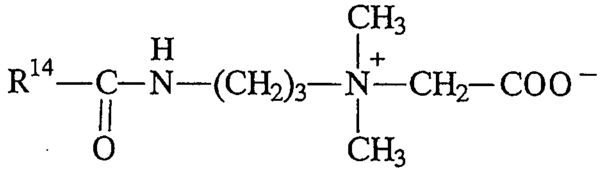

(12)

($R^{14}$: a group derived from coconut oil fatty acid)

In the fatty acid or its salt (c-3), $R^5$ is preferably a linear or branched alkyl or alkenyl group containing 9 to 17 carbon atoms on average, preferably 11 to 15 carbon atoms, more preferably 11 to 13 carbon atoms, from the viewpoint of foaming ability. As the fatty acid or its salt (c-3), mixed fatty acids derived from fat and oil materials may be used, and examples of fats and oils include coconut oil, palm kernel oil, rapeseed oil, etc. The fatty acids derived from fats and oils may be used alone, or a combination of several kinds of fatty acids derived from different fats and oils may be used, and when particularly a combination of fatty acids is used, the fatty acids are used desirably in such a ratio that the average number of carbon atoms in $R^5$ is in the range of 11 to 13.

$X^6$ represents a hydrogen atom, an alkali metal, $NH_4$ or alkanol ammonium, and examples of the alkali metal include lithium, sodium, potassium, etc., and examples of an alkanol amine forming an alkanol ammonium ion include mono-, di- or triethanolamine, mono-, di- or tripropanolamine, etc. $X^6$ is preferably sodium or potassium.

In the amine oxide (c-4), $R^6$ is an alkyl or alkenyl group containing 8 to 18 carbon atoms on average, preferably 10 to 14 carbon atoms, or an acyl amino alkyl group represented by $R^7CONH(CH_2)_b$- ($R^7CO$ represents an acyl group containing 8 to 18 carbon atoms on average, preferably 10 to 14 carbon atoms, and b is 2 to 4, preferably 3) from the viewpoint of detergency and foaming ability. $R^6$ and $R^7$ CO may be a mixed alkyl group or an alkenyl or acyl group derived from natural oils, for example, animal oils such as tallow and lard oil or vegetable oils such as soybean oil, coconut oil and palm kernel oil, or synthetic oils and mixed oils thereof. In particular, a mixed alkyl group or an acyl group derived from coconut oil and palm kernel oil is preferable.

Preferable amine oxides include lauryl dimethyl amine oxide represented by formula (13), lauroyl amino propyl dimethyl amine oxide represented by formula (14) and coconut oil fatty acid amide propyl dimethyl amine oxide represented by formula (15).

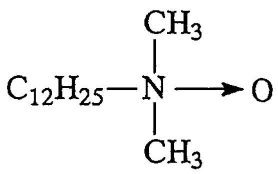

(13)

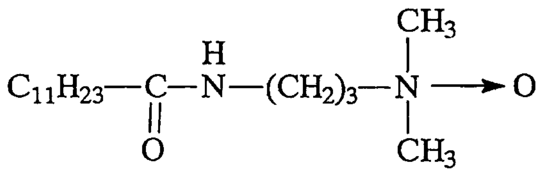

(14)

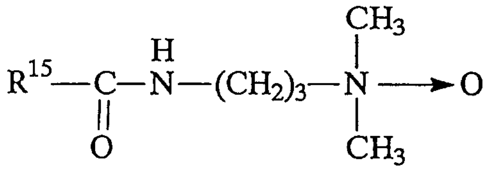

(15)

($R^{15}$: a group derived from coconut oil fatty acid)

In the isethionic acid-based surfactant (c-5), $R^8$ in the general formula (7) is preferably an alkyl or alkenyl group containing 11 to 13 carbon atoms on average. The alkali metal represented by Z includes lithium, sodium, potassium, etc., and the alkanol amine forming an alkanol ammonium ion includes mono-, di- or triethanolamine, mono-, di- or tripropanolamine, etc.

Preferable examples of the isethionic acid-based surfactant (c-5) include sodium lauroyl isethionate, sodium myristoyl isethionate, potassium lauroyl isethionate, potassium myristoyl isethionate, lauroyl isethionate triethanol ammonium, sodium cocoyl isethionate, potassium cocoyl isethionate, etc.

In the sugar-based surfactant (c-6), $R^9$ in the general formula (8) is preferably an alkyl group containing 8 to 18 carbon atoms on average, particularly 10 to 14 carbon atoms (decyl group, lauryl group, myristyl group, etc.). $R^{10}$ is preferably an alkylene group containing 2 to 3 carbon atoms. p is preferably 0 to 2, more preferably 0. G is a residue derived from a reducing sugar containing 5 to 6 carbon atoms, and the reducing sugar containing 5 to 6 carbon atoms is preferably glucose, galactose, fructose or the like. The average condensation degree (q) of the reducing sugar is 1 to 10, particularly preferably 1 to 4. q is determined preferably in consideration of physical properties derived from the number of carbon atoms in the alkyl or alkenyl group represented by $R^9$; for example, when $R^9$ is an alkyl or alkenyl group containing 8 to 11 carbon atoms, q is preferably 1 to 1.4, and when $R^9$ is an alkyl or alkenyl group containing 12 to 14 carbon atoms, q is preferably 1.5 to 4.0. q is determined by proton NMR.

In the alkanol amide (c-7), $R^{11}$ is a linear or branched alkyl or alkenyl group containing 7 to 17 carbon atoms on average, preferably 10 to 14 carbon atoms. $R^{12}$ is a hydrogen atom or a methyl group. $R^{13}$ represents a hydroxyl group or a hydrogen atom, and at least one of $(R^{13})r$ groups is a hydroxyl group. r is a number of 1 to 5, preferably 1 to 2.

Examples of the alkanol amide (c-7) include lauric acid monoethanol amide, coconut oil fatty acid monoethanol amide, palm kernel oil fatty acid monoethanol amide, lauric acid isopropanol amide, coconut oil fatty acid isopropanol amide, lauric acid glycerol amide, etc.

In the N-acylamino acid salt (C-8), the acyl group includes a saturated or unsaturated acyl group containing 8 to 18 carbon atoms on average, preferably 10 to 14 carbon atoms, for example a single acyl group such as octanoyl, decanoyl, lauroyl, myristoyl, palmitoyl, stearoyl or oleoyl. Preferably, the acyl group may be a mixed acyl group such as coconut oil fatty acid acyl or palm kernel oil fatty acid acyl.

Examples of the amino acid include glutamic acid, aspartic acid, glycine, sarcosine, alanine, β-alanine, N-methyl-β-alanine, etc. The salt includes, but is not limited to, alkali metal salts, alkaline earth metal salts, alkanol amine salts, etc., among which alkali metal salts such as sodium salts and potassium salts are preferable.

In the N-acyl-N-methyl taurine salt (C-9), the acyl group includes a saturated or unsaturated acyl group containing 8 to 18 carbon atoms on average, preferably 10 to 14 carbon atoms, for example, a single acyl group such as octanoyl, decanoyl, lauroyl, myristoyl, palmitoyl, stearoyl or oleoyl. Preferably, the acyl group may be a mixed acyl group such as coconut oil fatty acid acyl or palm kernel oil fatty acid acyl. The salt includes, but is not limited to, alkali metal salts, alkaline earth metal salts, alkanol amine salts, etc. Among them alkali metal salts such as sodium salts, potassium salts, etc. are preferable.

The glycol as component (d) used in the present invention is the one containing 5 to 6 carbon atoms, and includes, for example, dipropylene glycol, isoprene glycol, etc. These can be used as a mixture of two or more thereof, and are contained in an amount of preferably 0.1 to 30% by weight, more preferably 0.5 to 15% by weight, still more preferably 1 to 10% by weight, based on the whole composition.

The ratio by weight of the component (d) to the components (a) and (b), that is, (d)/((a) + (b)), is 95/5 to 5/95, preferably 80/20 to 20/80. Within this range, both foaming ability and low-temperature handling can be maintained.

Further, the total content of the components (a), (b) and (d) is 3 to 60% by weight, preferably 5 to 40% by weight. When the content is less than 3% by weight, the foaming ability is insufficient, while when the content is higher than 60% by weight, the viscosity is not stable at low temperatures.

As the component (e) used in the present invention, the thickening polymer compound having a carboxyl group is preferably the one having a carboxyl group in the molecule and having a crosslinked structure, and examples thereof include carboxyvinyl polymers having polyacrylic acid as main chain and an allyl sucrose structure or a pentaerythritol structure as a crosslinking group (commercial products such as Carbopol 940, Carbopol 941, Carbopol 980 and Carbopol 981 manufactured by B. F. Goodrich), acrylic acid/C10-30 alkyl methacrylate copolymers (commercial products such as Pemulen TR1, Pemulen TR2, Carbopol 1342, and Carbopol ETD2020 manufactured by B. F. Goodrich).

These can be used as a mixture of two or more thereof, and are contained in an amount of 0.005 to 5% by weight, preferably 0.1 to 2% by weight, based on the whole composition. A combination of the components (a) and (b) in the above range is preferable because the resulting cleanser composition can achieve both a freshening feeling in use and a suitable thickening effect.

The suitable viscosity of the cleanser composition preferable as a body or facial cleanser at 25°C is about 100 to 500,000 mPa·s, preferably 200 to 300,000 mPa·s, still more preferably 500 to 100,000 mPa·s.

When the cleanser composition of the present invention is used as a liquid composition such as shower gel or a body shampoo, the viscosity thereof at 25°C is preferably 200 to 4000 mPa·s, particularly 500 to 2000 mPa·s. When the cleanser composition of the present invention is formed into a paste such as a facial cleanser , the viscosity thereof at 25°C is preferably 10,000 to 300,000 mPa·s, particularly 20,000 to 100,000 mPa·s.

The cleanser composition of the present invention does not freeze even at low temperatures of 5°C or less with less increase in viscosity from ordinary temperatures to low temperatures.

The cleanser composition of the present invention can be compounded if necessary with other surfactants used in conventional cleansers, for example anionic surfactants such as alkyl benzene sulfonates, $\alpha$-olefin sulfonates, alkane sulfonates, $\alpha$-sulfofatty acid esters and polyoxy alkylene alkyl ether carboxylates, nonionic surfactants such as polyoxy alkylene alkyl ether and Pluronic surfactants, and cationic surfactants such as quaternary ammonium salts.

The cleanser composition of the present invention can be compounded if necessary with components used in conventional cleansers, for example, humectants such as propylene glycol, glycerine and sorbitol; viscosity regulators such as methyl cellulose, polyoxyethylene glycol distearate and ethanol; disinfectants such as triclosan and trichlorocarban; anti-inflammation agents such as potassium glycyrrhizate and acetic acid tocopherol; anti-dandruff agents such as zinc pyrithion and octopyrox; preservatives such as methylparaben, ethylparaben, propylparaben and butylparaben; besides oils, chelating agents, perfumes, coloring matters, feeling improvers, salts, pearlescent agents, sucrubbing agents, cooling agents, UV absorbing agents, plant extracts and antioxidants.

The cleanser composition of the present invention is suitable for washing the skin, hair, etc. with a low stimulus to the skin and a high foaming ability, and is useful as a facial cleanser, a shower gel, a shampoo, a body shampoo etc. Further, the cleanser composition of the present invention is useful as e.g. a cleanser such as a kitchen cleanser brought directly into contact with the skin for a long time.

## Examples

### Example 1

Using mixtures ab-1 to ab-3 consisting of components (a) and (b) shown in Table 1 and the component (c) shown in Table 2, cleanser compositions having the respective formulations shown in Tables 3 and 4 were produced in a usual manner. In the respective mixtures in Table 1, each of the content of trialkylphosphate was about 0.5% by weight. The pH value was regulated by an aqueous solution of phosphoric acid or sodium hydroxide.

The pH value of the resulting composition was measured in the following method to evaluate the foaming ability. The results are shown in Tables 3 and 4.

<Measurement of pH>

2 g cleanser composition was weighed, 38 g deionized water was added thereto and stirred for 5 minutes, and the pH of the uniform aqueous solution was measured with a pH meter (HORIBA pH meter F-22) at 25°C.

<Evaluation of foaming ability>

The cleanser composition was diluted 10-fold with deionized water, and 4 ml of the resulting aqueous solution was placed in each of 3 graduated 10 ml centrifuge tubes, and the centrifuge tubes were attached side by side to a commercial handless shaker (SHK-COCK manufactured by Asahi Techno Glass Co., Ltd.). The samples were shaken

at a temperature of 25°C in a shaking cycle of 180 reciprocating motions/min. at a shaking angle of 45° for a shaking time of 15 seconds. The amounts of foams in the respective samples were measured to give an average value of the 3 samples, and judged according to the following judgment criteria:

◎ 4.5 ml or more foams.
○: 3.5 ml to less than 4.5 ml foams.
Δ: 2.5 ml to less than 3.5 ml foams.
×: less than 2.5 ml foams.

Table 1

| | Starting alcohol ($R^1$-OH) | Average number of carbon atoms in $R^1$ | Branching degree of $R^1$ (%) | Average number of EO added (n) | Weight ratio of (a)/(b) | Counter ion | | |
|---|---|---|---|---|---|---|---|---|
| | | | | | | $X^1$ | $X^2$ | $X^3$ |
| ab-1 | Dobanol 23*1 | 12.5 | 25 | 0 | 80/20 | K | H | K |
| ab-2 | | | | 2 | 75/25 | Na | H | Na |
| ab-3 | | | | 2 | 60/40 | Na | H | Na |
| *1: a product of Mitsubishi Chemical Co.,Ltd. | | | | | | | | |

Table 2

| | |
|---|---|
| c-1-1 | Lauryl ethoxylate (2) sulfate (in formula(3), $R^2$=lauryl group, m=2, $X^4$=Na) |
| c-2-1 | Lauryl dimethyl hydroxy sulfobetaine (compound represented by formula (10)) |
| c-2-2 | Lauroyl amino propyl carboxy betaine (compound represented by formula (11)) |
| c-2-3 | Coco-amide propyl carboxy betaine (compound represented by formula (12)) |
| c-3-1 | Sodium laurate |
| c-4-1 | Lauryl dimethyl amine oxide |
| c-5-1 | Sodium lauroyl isethionate |
| c-5-2 | sodium coco-acyl isethionate |
| c-6-1 | Decyl glucoside |
| c-7-1 | Lauric acid monoethanol amide |
| c-8-1 | Sodium lauroyl glutamate |
| c-9-1 | Lauroyl-N-methyl taurine sodium salt |

Table 3

| | | | products of the present invention | | | | | | | | | | | | | |
|---|---|---|---|---|---|---|---|---|---|---|---|---|---|---|---|---|
| | | | 1 | 2 | 3 | 4 | 5 | 6 | 7 | 8 | 9 | 10 | 11 | 12 | 13 | 14 |
| Cleanser composition (weight %) | Mixture of component (a) and component (b) | ab−1 | 15 | 20 | | | | | | | | | | | | |
| | | ab−2 | | | 20 | 15 | 15 | 15 | 15 | 15 | 15 | 15 | 15 | 15 | 15 | 15 |
| | | ab−3 | | | | | | | | | | | | | | |
| | component (c) | c-1-1 | 5 | | | 5 | | | | | | | | | | |
| | | c-2-1 | | | | | 5 | | | | | | | | | |
| | | c-2-2 | | | | | | 5 | | | | | | | | |
| | | c-2-3 | | | | | | | 5 | | | | | | | |
| | | c-3-1 | | | | | | | | 5 | | | | | | |
| | | c-4-1 | | | | | | | | | 5 | | | | | |
| | | c-5-1 | | | | | | | | | | 5 | | | | |
| | | c-6-1 | | | | | | | | | | | 5 | | | |
| | | c-7-1 | | | | | | | | | | | | 5 | | |
| | | c-8-1 | | | | | | | | | | | | | 5 | |
| | | c-9-1 | | | | | | | | | | | | | | 5 |
| | pH regulating agent (aqueous solution of phosphoric acid or sodium hydroxide) | | Suitable amount | | | | | | | | | | | | | |
| | Deionized water | | Balance (amount for adjustment to 100% by weight in total) | | | | | | | | | | | | | |
| Evaluation result | pH | | 5.5 | 5.5 | 5.5 | 5.5 | 5.5 | 5.5 | 5.5 | 5.5 | 5.5 | 5.5 | 5.5 | 5.5 | 5.5 | 5.5 |
| | Foaming ability | | ○ | ○ | ○ | ○ | ◎ | ○ | ◎ | ○ | ○ | ◎ | ◎ | ○ | ○ | ○ |

Table 4

| Cleanser composition (weight %) | | Comparative product 1 | 2 | 3 | 4 | 5 | 6 | 7 | 8 | 9 | 10 | 11 | 12 | 13 |
|---|---|---|---|---|---|---|---|---|---|---|---|---|---|---|
| Mixture of component (a) and componet (b) | ab－1 | | 15 | | | | | | | | | | | |
| | ab－2 | | | 15 | 15 | 15 | 15 | 15 | 15 | 15 | 15 | 15 | 15 | 15 |
| | ab－3 | 15 | | | | | | | | | | | | |
| Component (c) | c－1－1 | 5 | 5 | 5 | | | | | | | | | | |
| | c－2－1 | | | | 5 | | | | | | | | | |
| | c－2－2 | | | | | 5 | | | | | | | | |
| | c－2－3 | | | | | | 5 | | | | | | | |
| | c－3－1 | | | | | | | 5 | | | | | | |
| | c－4－1 | | | | | | | | 5 | | | | | |
| | c－5－1 | | | | | | | | | 5 | | | | |
| | c－6－1 | | | | | | | | | | 5 | | | |
| | c－7－1 | | | | | | | | | | | 5 | | |
| | c－8－1 | | | | | | | | | | | | 5 | |
| | c－9－1 | | | | | | | | | | | | | 5 |
| pH regulating agent (aqueous solution of phosphoric acid or sodium hydroxide) | | Suitable amount | | | | | | | | | | | | |
| Deionized water | | Balance (amount for adjustment to 100% by weight in total) | | | | | | | | | | | | |
| Result of evaluation | pH | 5.5 | 7.0 | 7.0 | 7.0 | 7.0 | 7.0 | 7.0 | 7.0 | 7.0 | 7.0 | 7.0 | 7.0 | 7.0 |
| | Foaming ability | △ | △ | △ | △ | △ | △ | △ | × | △ | △ | × | △ | △ |

Example 2

Using a phosphate mixture shown in Table 5, a cleanser composition having a formulation shown in Table 6 was produced in a usual manner. The resulting cleanser composition was evaluated for its viscosity at 0°C and 25°C and for its foaming ability at 40°C. Assuming use of the cleanser composition in washing of the face and body with warm water, the foaming ability was evaluated at 40°C. The results are shown collectively in Table 6.

(Evaluation method)

(1) Viscosity

Each cleanser composition was stored at each temperature of 0°C and 25°C for 24 hours, and the viscosity at each temperature was measured with a BM type viscometer (TOKIMEC INC.).

(2) Foaming ability

Each cleanser composition was diluted 10-fold with deionized water, and 2 g lanoline was added as artificial dirt to 100 ml of the resulting aqueous solution, then introduced into a graduated glass cylinder having an inner diameter of 6.5 cm, and stirred at 40°C at 1000 rpm for 1 minute by contrarotation at 10-second intervals with a flat propeller. Five minutes after stirring was finished, the amount of foams generated was measured and judged according to the following criteria:

○: 155 ml or more foams.
Δ: 145 ml to less than 155 ml foams.
×: less than 145 ml foams.

Table 5

| Phosphate | Starting alcohol ($R^1$-OH) | Avereage number of carbon atoms in $R^1$ | Branching degree of $R^1$ (%) | Average number of moles of EO added (n) | weight ratio of (a)/(b) | Counter ion | | |
|---|---|---|---|---|---|---|---|---|
| | | | | | | $X^1$ | $X^2$ | $X^3$ |
| ab-4 | Diadol 11 *1 | 11.0 | 50 | 0 | 80/20 | K | H | K |
| ab-3 | Dobanol 23*1 | 12.5 | 25 | 2 | 60/40 | Na | H | Na |
| ab-2 | Dobanol 23 *1 | 12.5 | 25 | 2 | 75/25 | Na | H | Na |
| ab-5 | Dobanol 23 *1 | 12.5 | 25 | 2 | 95/5 | Na | H | Na |
| ab-6 | Kalcol 2098*1 | 12.0 | 0 | 2 | 75/25 | Na | H | Na |
| *1 : product of Mitsubishi Chemical Co., Ltd.<br>*2 : product of Kao corporation | | | | | | | | |

Table 6

| Component (weight %) | | Products of the present invention 1 | 2 | 3 | 4 | Comparative product 1 | 2 | 3 | 4 | 5 | 6 | 7 | 8 | 9 |
|---|---|---|---|---|---|---|---|---|---|---|---|---|---|---|
| A·B | Phosphate (ab−4) | 30 | 30 | | | 30 | | | | 30 | | | | |
| | Phosphate (ab−3) | | | | | | 30 | | | | 30 | | | |
| | Phosphate (ab−2) | | | 30 | 30 | | | | | | | 30 | | |
| | Phosphate (ab-5) | | | | | | | 30 | | | | | 30 | |
| | Phosphate (ab-6) | | | | | | | | 30 | | | | | 30 |
| C | Dipropylene glycol | 5 | | 5 | | | 5 | 5 | 5 | | | | | |
| | Isoprene glycol | | 5 | | 5 | | | | | | | | | |
| | 1,3-Butanediol | | | | | 5 | | | | | | | | |
| | Deionized water | Balance | | | | | | | | | | | | |
| Viscosity (mPa·s) : 0°C | | 606 | 590 | 1260 | 1110 | 2530 | 3180 | 2060 | 778 | 3240 | 2600 | 5160 | 5800 | 5880 |
| Viscosity (mPa·s) : 25°C | | 292 | 230 | 718 | 690 | 254 | 908 | 996 | 202 | 366 | 870 | 1220 | 1180 | 334 |
| Foaming ability | | ○ | ○ | ○ | ○ | △ | × | × | × | △ | × | × | × | × |

Example 3 (Facial cleanser)

A facial cleanser in a paste form having the formulation shown below was produced in a usual manner.

The resulting facial cleanser had a high foaming ability and a viscosity of 45, 000 mPa·s at 25°C and a viscosity of 48, 000 mPa·s at 0°C with less increase in viscosity at low temperatures.

(Components)

| | |
|---|---|
| Phosphate mixture (ab-2) | 20 (weight %) |
| Lauryl phosphoric acid | 5 |
| Lauryl hydroxy sulfobetaine (c-2-1) | 3 |
| Lauric acid | 2 |
| Dipropylene glycol | 3 |
| Ethylene glycol distearate | 1.5 |
| Dibutyl hydroxy toluene | 0.02 |
| EDTA·4$H_2O$ | 0.02 |
| Sodium chloride | 3 |
| Perfume | 0.5 |
| 48% Sodium hydroxide | amount for adjustment to pH 5.5 |
| Water | balance |

Example 4

Using a phosphate mixture shown in Table 5, a cleanser composition having a formulation shown in Table 7 was produced in a usual manner. The resulting cleanser composition was evaluated for rinsing and viscosity. The results are shown collectively in Table 7.

(Evaluation method)

(1) Rinsing

An evaluation panel of 10 men and 10 women took 5 g of each cleanser composition on one hand, diluted it with tap water, and washed both bands, and easiness of rinsing was evaluated according to the following criteria and judged in terms of average score.

<Evaluation criteria>

Score 0: Hardly rinsed.
Score 1: Slightly hardly rinsed.
Score 2: Easily rinsed.
Score 3: Very easily rinsed.

<Judgment criteria>

○: An average score of 2 to 3.
Δ: An average score of 1 to less than 2.
×: An average score of 0 to less than 1.

(2) Viscosity

The viscosity of each cleanser composition at 25°C was measured with a BM type viscometer (TOKIMEC INC.).

Table 7

| Component (weight-%) | | Present invention | | | | | | Comparative product | | | | | | | | | | |
|---|---|---|---|---|---|---|---|---|---|---|---|---|---|---|---|---|---|---|
| | | 1 | 2 | 3 | 4 | 5 | 6 | 1 | 2 | 3 | 4 | 5 | 6 | 7 | 8 | 9 | 10 | 11 |
| AB | Phosphate (ab−4) | 15 | 15 | 15 | | | | | | | | | | 15 | | | | |
| | Phosphate (ab−3) | | | | | | | | | | | 15 | 15 | | 15 | | | |
| | Phosphate (ab−2) | | | | 15 | 15 | 15 | | | | | | | | | 15 | | |
| | Phosphate (ab-5) | | | | | | | 15 | 15 | | | | | | | | 15 | |
| | Phosphate (ab-6) | | | | | | | | | 15 | 15 | | | | | | | 15 |
| C | Carbopol 940 | 1.0 | | | 1.0 | | | | | | | | | | | | | |
| | Carbopol ETD2020 | | 1.0 | | | 1.0 | | 1.0 | | 1.0 | | 1.0 | | | | | | |
| | Pemulen TR1 | | | 1.0 | | | 1.0 | | 1.0 | | 1.0 | | 1.0 | | | | | |
| | Malic acid | Amount for adjustment to predetermined pH | | | | | | | | | | | | | | | | |
| | Deionized water | Balance | | | | | | | | | | | | | | | | |
| pH | | 5.6 | 5.4 | 5.5 | 5.4 | 5.5 | 5.5 | 5.5 | 5.5 | 5.5 | 5.6 | 5.5 | 5.4 | 5.5 | 5.5 | 5.4 | 5.5 | 5.5 |
| Rinsing | | ○ | ○ | ○ | ○ | ○ | ○ | △ | △ | △ | △ | △ | △ | △ | × | △ | △ | △ |
| Viscosity (mPa·s) | | 6090 | 5930 | 3600 | 6680 | 7030 | 3970 | 5500 | 2060 | 3610 | 2220 | 4060 | 3130 | 39.2 | 39.0 | 80.6 | 62.8 | 44.2 |

Example 5 (Body shampoo)

A body shampoo having the formulation shown below was produced in a usual manner.

The resulting body shampoo had a viscosity of 3, 100 mPa·s at 25°C and could be easily rinsed to give a freshening feeling in use.

| (Components) | |
|---|---|
| Phosphate mixture (ab-2) | 15 (weight %) |
| Pemulen TR1 | 0.2 |
| Sodium lauryl ether sulfate | 3 |
| Lauryl hydroxy sulfobetaine (c-2-1) | 3 |
| Lauric acid | 1 |
| Glycerine | 10 |
| Ethylene glycol distearate | 2 |
| Dibutyl hydroxy toluene | 0.02 |
| EDTA·4$H_2O$ | 0.02 |
| 85% Phosphoric acid | amount for adjustment to pH 5.5 |
| Perfume | 0.8 |
| Water | balance |

Example 6 (Facial cleanser)

A facial cleanser in a paste form having the formulation shown below was produced in a usual manner.

The resulting facial cleanser had a viscosity of 83, 000 mPa·s at 25°C and could be easily rinsed to give a freshening feeling in use.

| (Components) | |
|---|---|
| Phosphate mixture (ab-2) | 15 (weight %) |
| Carbopol ETD2020 | 0.6 |
| Laurylphoshoric acid | 5 |
| Lauryl hydroxy sulfobetaine (c-2-1) | 5 |
| Lauric acid | 1 |
| Glycerine | 10 |
| Ethylene glycol distearate | 1.5 |
| Dibutyl hydroxy toluene | 0.02 |
| EDTA·4$H_2O$ | 0.02 |
| 85% Phosphoric acid | amount for adjustment to pH 5.5 |
| Perfume | 0.8 |
| Water | balance |

Example 7

Cleanser compositions were prepared using ab-2 shown in Table 1 and component (c) of a co-surfactant shown in Table 2 according to the formulations shown in Table 8. They were tested in the same way as shown in Example 1. Results of foaming ability (ml) are shown in Table 8. It is noted that the composition obtained by combining the phosphate mixture of the invention with a co- surfactant is significantly improved in view of foaming ability.

Table 8

| | | Present invention | | | | | | | | | | | | | | | |
|---|---|---|---|---|---|---|---|---|---|---|---|---|---|---|---|---|---|
| | | 41 | 42 | 43 | 44 | 45 | 46 | 47 | 48 | 49 | 50 | 51 | 52 | 53 | 54 | 55 | 56 |
| Mixture of component(a) +component(b) | ab−2 | 1.6 | 1.6 | 1.6 | 1.6 | 1.6 | 2.0 | 2.0 | 4.0 | 4.0 | 4.0 | 6.0 | 6.0 | 7.0 | 7.0 | 9.0 | 9.0 |
| Component (c) | c−1−1 | 0.4 | | | | | | | | | | | | | | | |
| | c−2−1 | | 0.4 | | | | | | | | | | | | | | |
| | c−2−2 | | | | | | | | 2.0 | | | | | | | | |
| | c−3−1 | | | 0.4 | | | | | | | | | | | | | |
| | c−4−1 | | | | 0.4 | | | | | | | | | | | | |
| | c−5−2 | | | | | 0.4 | | | | | | | | | | | |
| | c−6−1 | | | | | | | | | | | | | 2.0 | | | |
| | c−7−1 | | | | | | | | | 2.0 | | | | | | | |
| | c−8−1 | | | | | | | | | | 2.0 | | | | | | |
| | c−9−1 | | | | | | | | | | | | | | 2.0 | | |
| pH adjuster | | Proper amount | | | | | | | | | | | | | | | |
| Deionized water | | Balance | | | | | | | | | | | | | | | |
| pH | | 5.5 | 5.5 | 5.5 | 5.5 | 5.5 | 5.5 | 7.0 | 5.5 | 5.5 | 5.5 | 5.5 | 7.0 | 5.0 | 5.0 | 5.5 | 7.0 |
| Amount of foams (ml) | | 3.7 | 4.5 | 5.7 | 4.7 | 4.9 | 3.8 | 3.5 | 4.5 | 4.3 | 4.8 | 3.6 | 3.5 | 4.4 | 4.5 | 3.8 | 3.7 |

Example 8 (Facial cleanser)

A facial cleanser formulation in a paste form is shown below.

| (Components) | |
|---|---|
| Phosphate mixture (ab-2) | 15 (weight %) |
| Carbopol ETD2020 | 0.6 |
| Lauroylaminopropyl carbobetaine (c-2-2) | 5 |
| Sorbitol | 14 |
| Dibutyl hydroxy toluene | 0.02 |
| EDTA-$4H_2O$ | 0.02 |
| 85% Phosphoric acid | amount for adjustment to pH 5.5 |
| Perfume | 0.4 |
| Water | balance |

## Claims

1. A cleanser composition which is weakly acidic and comprises the following components (a) and (b) in a (a)/(b) ratio of from 65/35 to 90/10 by weight:

   (a) a phosphate monoester represented by the general formula (1) or a salt thereof:

   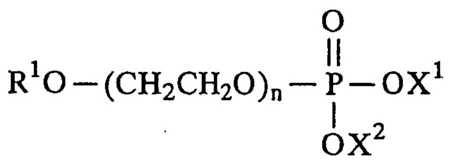

   (1)

   wherein $R^1$ represents an alkyl or alkenyl group containing 9 to 15 carbon atoms on average with a branching degree of 10% or more, $X^1$ and $X^2$ each represent a hydrogen atom or an alkali metal, and n is a number of 0 to 5 which refers to the number of ethylene oxide units added on average,
   (b) a phosphate diester represented by the general formula (2) or a salt thereof:

   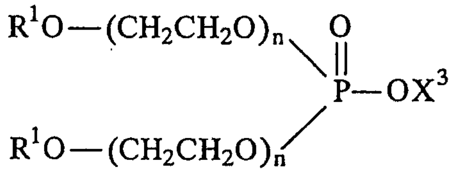

   (2)

   wherein $R^1$ and n each have the same meaning as defined above, and $X^3$ represents a hydrogen atom or an alkali metal.

2. The cleanser composition according to claim 1, which exhibits a pH value of 4.5 to 6. 5 upon dilution at a concentration of 5% by weight with deionized water.

3. The cleanser composition according to claim 1 or 2, wherein the total amount of the components (a) and (b) is 3 to 50% by weight.

4. The cleanser composition according to claim 1, which further comprises at least one co-surfactant (referred to hereinafter as component (c)) selected from the group consisting of an alkyl ethoxylate sulfate, a betaine-type surfactant, a fatty acid or a salt thereof, an amine oxide, an isethionic acid-based surfactant, a sugar-based surfactant, an alkanol amide, an N-acylamino acid salt and an N-acyl-N-methyl taurine salt.

**5.** The cleanser composition according to claim 4, which comprises, as said component (c), at least one member selected from the group consisting of the following (c-1) to (c-9):

(c-1) an alkyl ethoxylate sulfate represented by the general formula (3):

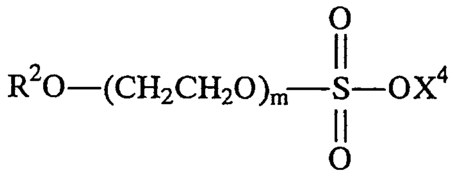 (3)

wherein $R^2$ represents a linear or branched alkyl or alkenyl group containing 10 to 18 carbon atoms on average, $X^4$ represents an alkali metal, and m is a number of 0 to 10 indicating the number of ethylene oxide units added on average.

(c-2) a betaine-type surfactant represented by the general formula (4):

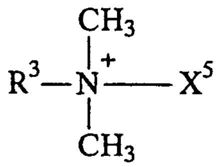 (4)

wherein $R^3$ represents an alkyl or alkenyl group containing 8 to 18 carbon atoms on average or an acyl amino alkyl group represented by the formula $R^4CONH(CH_2)_a$- whereupon $R^4CO$ represents an acyl group containing 8 to 18 carbon atoms on average and a is an integer of 2 to 4, and $X^5$ represents a $-CH_2CH(OH)CH_2SO_3^-$ group or a $-CH_2COO^-$ group.

(c-3) a fatty acid or a salt thereof represented by the general formula (5):

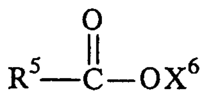 (5)

wherein $R^5$ represents a linear or branched alkyl or alkenyl group containing 9 to 17 carbon atoms on average, and $X^6$ represents a hydrogen atom, an alkali metal, $NH_4$ or alkanol ammonium.

(c-4) an amine oxide represented by the general formula (6):

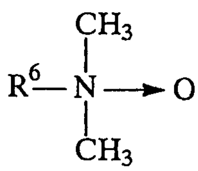 (6)

wherein $R^6$ represents a linear or branched alkyl or alkenyl group containing 8 to 18 carbon atoms on average or an acyl amino alkyl group represented by the formula $R^7CONH(CH_2)_b$- whereupon $R^7CO$ represents an acyl group containing 8 to 18 carbon atoms on average and b is an integer of 2 to 4.

(c-5) an isethionic acid-based surfactant represented by the general formula (7):

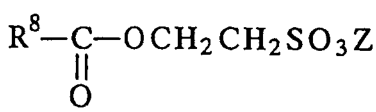 (7)

wherein $R^8$ represents a linear or branched alkyl or alkenyl group containing 9 to 17 carbon atoms on average, and Z represents a hydrogen atom, an alkali metal, $NH_4$ or alkanol ammonium.
(c-6) a sugar-based surfactant represented by the general formula (8):

$$R^9\text{-}O\text{-}(R^{10}O)_p\text{-}(G)_q \qquad (8)$$

wherein $R^9$ represents a linear or branched alkyl or alkenyl group containing 8 to 18 carbon atoms on average, $R^{10}$ represents an alkylene group containing 2 to 4 carbon atoms, G represents a residue derived from a reducing sugar containing 5 to 6 carbon atoms, p is a number of 0 to 10 indicating the number of alkylene oxide units added on average, and q is a number of 1 to 10 indicating the average condensation degree of the reducing sugar.
(c-7) an alkanol amide represented by the general formula (9):

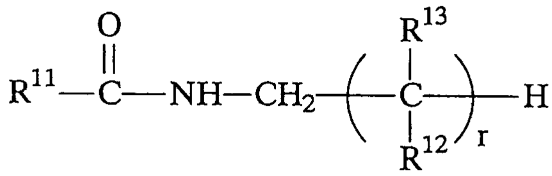

(9)

wherein $R^{11}$ represents a linear or branched alkyl or alkenyl group containing 7 to 17 carbon atoms on average, $R^{12}$ represents a hydrogen atom or a methyl group, $R^{13}$ represents a hydroxyl group or a hydrogen atom, r is a number of 1 to 5, and $(R^{12})_r$ groups and $(R^{13})r$ groups may be the same or different, respectively, provided that one of $(R^{13})r$ groups is a hydroxyl group.
(c-8) an N-acylamino acid salt having an acyl group containing 8 to 18 carbon atoms on average, and
(c-9) an N-acyl-N-methyl taurine salt having an acyl group containing 8 to 18 carbon atoms on average.

**6.** The cleanser composition according to claim 4 or 5, wherein the content of the component (c) is 0. 5 to 20% by weight.

# INTERNATIONAL SEARCH REPORT

International application No.
PCT/JP03/12941

A. CLASSIFICATION OF SUBJECT MATTER
Int.Cl$^7$ C11D1/34, 1/29, 1/90, 1/92, 1/75, 9/02, 1/28, 1/72, A61K7/50

According to International Patent Classification (IPC) or to both national classification and IPC

B. FIELDS SEARCHED

Minimum documentation searched (classification system followed by classification symbols)
Int.Cl$^7$ C11D1/00-19/00, A61K7/00-7/50

Documentation searched other than minimum documentation to the extent that such documents are included in the fields searched

Electronic data base consulted during the international search (name of data base and, where practicable, search terms used)
CAPLUS(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | JP 2001-181677 A (KAO CORP.), 03 July, 2001 (03.07.01), Abstract; Claim 1; Par. Nos. [0018], [0020]; examples 1 to 11; comparative examples 1 to 8 (a reference cited in the specification of the present application) & CN 1303917 A | 1-6 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 26 December, 2003 (26.12.03) | 27 January, 2004 (27.01.04) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP03/12941

C (Continuation). DOCUMENTS CONSIDERED TO BE RELEVANT

| Category* | Citation of document, with indication, where appropriate, of the relevant passages | Relevant to claim No. |
|---|---|---|
| X | JP 4-211009 A (UNILEVER PLC.), 03 August, 1992 (03.08.92), Abstract; Claims 1 to 2, 4, 8 to 12; Par. Nos. [0023] to [0029], [0032] to [0090]; examples 1 to 14<br>& EP 442701 A2 & US 5139781 A1<br>& GB 9003199 A & DE 69118944 C<br>& CA 2035984 A & KR 9614786 B<br>& IN 172847 A & AT 137111 E<br>& ZA 9101067 A & AU 9170867 A1<br>& BR 9100584 A | 1-6 |
| X | JP 2-193910 A (UNILEVER PLC.), 31 July, 1990 (31.07.90), Claims 1, 10, 15 to 18; page 8, upper left column, line 1 to page 11, lower right column, line 13; examples 1 to 24<br>& EP 371803 A1 & US 5180879 A1<br>& GB 8828017 A & DE 68903474 D<br>& CA 2003843 A & KR 9307914 B<br>& AU 8945580 A1 & AT 82117 T<br>& ES 2052935 T & ZA 8909199 A<br>& BR 8906120 A | 1-6 |
| X | JP 7-48244 A (KAO CORP.), 21 February, 1995 (21.02.95), Abstract; Claim 1; Par. No. [0021]; examples 1 to 4<br>(Family: none) | 1-6 |
| E,X | JP 2003-313585 A (KAO CORP.), 06 November, 2003 (06.11.03), Abstract; Claims 1 to 3; Par. No. [0023]; example 1<br>(Family: none) | 1-6 |
| E,X | JP 2003-313586 A (KAO CORP.), 06 November, 2003 (06.11.03), Abstract; Claims 1 to 3; Par. Nos. [0019] to [0021], [0024]; example 1<br>(Family: none) | 1-6 |
| E,X | JP 2003-313587 A (KAO CORP.), 06 November, 2003 (06.11.03), Abstract; Claims 1 to 3; Par. No. [0023]; example 1<br>(Family: none) | 1-6 |
| E,X | JP 2003-313588 A (KAO CORP.), 06 November, 2003 (06.11.03), Abstract; Claims 1 to 3; Par. Nos. [0018] to [0020], [0023]; example 1<br>(Family: none) | 1-6 |

Form PCT/ISA/210 (continuation of second sheet) (July 1998)

**INTERNATIONAL SEARCH REPORT**

| International application No. |
| --- |
| PCT/JP03/12941 |

C (Continuation). DOCUMENTS CONSIDERED TO BE RELEVANT

| Category* | Citation of document, with indication, where appropriate, of the relevant passages | Relevant to claim No. |
| --- | --- | --- |
| E,X | JP 2003-336091 A (KAO CORP.), 28 November, 2003 (28.11.03), Abstract; Claims 1 to 4; Par. No. [0015]; examples 1 to 18 & CN 1443837 A | 1-6 |

Form PCT/ISA/210 (continuation of second sheet) (July 1998)